# EUROPEAN PATENT SPECIFICATION

(11) **EP 4 210 690 B1**
(45) Date of publication and mention of the grant of the patent: **29.01.2025**
(21) Application number: 21786063.4
(22) Date of filing: 03.09.2021
(51) Int. Cl.: A61K 31/27, A61K 31/407, A61K 31/4178, A61K 31/439, A61K 31/522, A61P 27/10, A61P 27/02, A61K 9/00

(54) **COMPOSITIONS AND METHODS FOR TREATING PRESBYOPIA, HYPEROPIA, ASTIGMATISM, DECREASED STEREOPSIS, AND DECREASED CONTRAST SENSITIVITY**
ZUSAMMENSETZUNGEN UND VERFAHREN ZUR BEHANDLUNG VON PRESBYOPIE, HYPEROPIE, ASTIGMATISMUS, VERRINGERTER STEREOPSIS UND VERMINDERTER KONTRASTEMPFINDLICHKEIT
COMPOSITIONS ET MÉTHODES DE TRAITEMENT DE LA PRESBYTIE, DE L'HYPERMÉTROPIE, DE L'ASTIGMATISME, D'UNE DIMINUTION DE LA STÉRÉOPSIE ET D'UNE DIMINUTION DE LA SENSIBILITÉ AU CONTRASTE

(30) Priority: 11.09.2020 US 202063077142 P
(43) Date of publication of application: 19.07.2023
(73) Proprietor: Intratus-Nevada, Inc., Henderson, NV 89044 (US)
(72) Inventor: NANDURI, Padma, La Jolla, California 92037 (US); DYER, Aaron, La Jolla, California 92037 (US)
(74) Representative: Müller-Boré & Partner Patentanwälte PartG mbB
(86) International application number: PCT/US2021/048985
(87) International publication number: WO 2022/055796

(56) References cited:
- WO-A1-2020/033714
- WO-A2-00/64425
- WO-A2-2011/053792
- WO-A2-98/30900
- US-A1- 2011 152 274
- US-A1- 2016 008 278
- US-A1- 2019 321 337
- US-B1- 6 410 544
- US-B1- 9 867 810
- US-B2- 9 034 830

## Description

### TECHNICAL FIELD

The present disclosure relates generally to compositions for treatment of impairec visual acuity caused by presbyopia, hyperopia, or astigmatism and compositions for enhancing contrast sensitivity and stereopsis.

### BACKGROUND OF THEDISCLOSURE

Presbyopia is the leading cause of visual disability in otherwise healthy eyes. It is an age-related process in which a gradual thickening and loss of flexibility of the natural lens of the eye occurs.

These age-related changes occur within the proteins in the lens, making the lens harder and less elastic over time. Age-related changes also take place in the muscle fibers surrounding the lens. With less elasticity, it gets difficult for the eyes to focus on close objects.

Young, properly functioning eyes are able to see at near distances, an ability that deteriorates with aging. Presbyopia normally develops as a person ages and is associated with a natural progressive loss of accommodation. A presbyopic eye loses the ability to rapidly and easily focus on objects at near distances.

The most common treatment for presbyopia is the use of over-the-counter reading glasses. Reading glasses allow the eye to focus on near objects and maintain a clear image. This approach is similar to that of treating hyperopia, or farsightedness.

Contact lenses and intra-ocular lenses (IOLs) have also been used to treat presbyopia, for example, by relying on monovision (where one eye is corrected for distance-vision, while the other eye is corrected for near-vision) or bilateral correction with either bi-focal or multi-focal lenses. Laser ablation has also been used to treat presbyopia. These procedures seek to correct the problem for long term purposes using drastic steps (surgery, laser ablation, etc.) or require wearing corrective lenses.

Hyperopia or farsightedness is a common vision condition in which distant objects are seen clearly, but objects nearby may be blurry. The degree of farsightedness influences the eye's ability to focus. People with severe farsightedness have blurry vision for both near and far objects, while those with mild farsightedness generally have blurry vision at a distance and severely blurry vision for closer objects.

Hyperopia usually is present at birth and tends to run in families. Hyperopia is easily corrected with eyeglasses or contact lenses. Another treatment option is surgery.

Astigmatism is a type of refractive error in which the eye does not focus light evenly on the retina, resulting in distorted or blurred vision at any distance. Other symptoms can include eye strain, headaches, and difficulty driving at night. If astigmatism occurs in early life, it can later result in amblyopia. The cause of astigmatism is not clear, however, it is believed to be partly related to genetic factors. The underlying mechanism involves an irregular curvature of the cornea or abnormalities in the lens of the eye.

Astigmatism may be corrected with eyeglasses, contact lenses, or refractive surgery. Glasses are the simplest and safest, although contact lenses can provide a wider field of vision. Refractive surgery can eliminate the need to wear corrective lenses altogether by permanently changing the shape of the eye but, like all elective surgery, comes with both greater risk and expense than the non-invasive options.

Contrast sensitivity is the visual ability to distinguish an object from its background; it is not the same as visual acuity. Poor contrast sensitivity is common in many visual disorders including cataract, diabetic retinopathy and many retinal disorders. Contrast sensitivity in these patients is generally treated with the use of high contrast filters, better lighting and the use of bolder higher contrast materials. In cataract patients, surgical removal of the cataract may significantly reduce contrast sensitivity.

Stereopsis refers to the perception of depth and 3-dimensional structure obtained on the basis of visual information deriving from two eyes by individuals with normally developed binocular vision.

The most common cause for loss of stereoscopic vision is decreased near or far visual acuity due to refractive error. Weakened binocular vision due to strabismus (deviating eye) or amblyopia, in which one eye has failed to form an adequate input to the visual cortex and anisometropia are other less common causes of stereopsis.

Photopic vision is the vision of the eye under well-lit conditions (luminance level 10 to 10⁸ cd/m²). In humans and many other animals, photopic vision allows color perception, mediated by cone cells, and a significantly higher visual acuity and temporal resolution than available with scotopic vision. Most older adults lose photopic spatial contrast sensitivity. Adults in their 70s require about three times more contrast to detect high spatial frequencies than adults in their 20s.

Scotopic vision is the vision of the eye under low-light levels (luminance level 10⁻⁶ to 10^{-3.5} cd/m²). In the human eye, cone cells are nonfunctional in low visible light. Scotopic vision is produced exclusively through rod cells, which are most sensitive to wavelengths of around 498 nm (green-blue) and are insensitive to wavelengths longer than about 640 nm (reddish orange). This condition is called the Purkinje effect.

Visual adaptation is much faster under photopic vision; for example, it can occur in 5 minutes for photopic vision but it can take 30 minutes to transition from photopic to scotopic.

Mesopic vision depends on both the external light level and the speed of the biochemical processes providing the energy to turn the rod- system fully on. Problems in mesopic vision are related to a pathological transition from photopic to scotopic vision and may present clinically as night blindness. There currently is no approved pharmaceutical therapy available to correct presbyopia, hyperopia, astigmatism or poor contrast sensitivity or decreased stereopsis. Thus, there remains a need for ameliorating or reducing these vision conditions for patients that do not wish to undergo surgery or use corrective glasses or contact lenses or alter their environment to accommodate their lack of visual acuity.

WO 2020/033714 discloses an ophthalmic composition comprising a cholinesterase inhibitor (physostigmine) and a miotic agent for use in the treatment of presbyopia by topical administration.

US 2019/321337, US 6 410 544 B1, US 2011/152274 and WO 00/64425 disclose the use of the miotic agents pilocarpine, carbachol, cevimeline or physostigmine in the treatment of presbyopia, hyperopia and/or astigmatism. The agents are formulated for topical administration to the eye.

### SUMMARY OF THE DISCLOSURE

Note that the references to methods of treatment in the summary and detailed description of the invention in this description are to be interpreted as references to the compounds, pharmaceutical compositions and medicaments of the present invention for use in a method for treatment of the human (or animal) body by therapy.

In one aspect of the disclosure there is provided a method of treating impaired visual acuity, decreased contrast sensitivity or decreased stereopsis in a subject in need thereof, comprising applying a topical composition comprising a methylated xanthine and an ophthalmic miotic agent to at least one eyelid of the subject, wherein the visual acuity, contrast sensitivity, stereopsis, or a combination thereof is improved. In certain embodiments the impaired visual acuity is presbyopia, hyperopia, or astigmatism.

In embodiments of this aspect, the treatment of impaired visual acuity, contrast sensitivity or decreased stereopsis results in improvement of the at least one parameter of vision for at least 4 hours, at least 5 hours, at least 6 hours, at least 7 hours, at least 8 hours, at least 9 hours, at least 10 hours, at least 12 hours, or longer. In certain embodiments, the composition is applied to at least one eyelid at least once daily or at least twice per day. In certain embodiments, the composition is applied to at least one eyelid of both eyes of the subject.

In certain embodiments, the methylated xanthine is caffeine, theophylline, dyphylline, theobromine, aminophylline, pentoxyphylline, or a pharmaceutically acceptable salt thereof. In certain embodiments the ophthalmic miotic agent is a cholinergic agent which is pilocarpine, carbachol, cevimeline or physostigmine or a pharmaceutically acceptable salt thereof. In certain embodiments, the composition comprises a methylated xanthine selected from caffeine, theophylline, dyphylline, theobromine, aminophylline, pentoxyphylline, and pharmaceutically acceptable salts thereof and a cholinergic agent selected from pilocarpine, carbachol, cevimeline, physostigmine and pharmaceutically acceptable salts thereof. In certain embodiments, the composition that is applied to the eyelid comprises from 0.05 to 10 wt% methylated xanthine. In certain embodiments the composition comprises about 0.05 to 1 wt% methylated xanthine, such as caffeine, and about 3 to 8 wt% pilocarpine, 1 to 3 wt% carbachol, 0.25 to 3 wt% physostigmine, or 4 to 15 wt% cevimeline.

In another aspect of the disclosure, there is provided a method for treating presbyopia, hyperopia, astigmatism, decreased contrast sensitivity, or decreased stereopsis and lowering incidence of at least one adverse event associated with application of an ophthalmic miotic agent to the eye of a subject, said method comprising applying a topical composition comprising a 0.05 to 10 wt% methylated xanthine in combination with the ophthalmic miotic agent to an outer eyelid of the subject. In certain embodiments, the one or more adverse effects are selected from the group consisting of ciliary spasm, ciliary induced brow ache, ciliary induced headache, eye redness, blurry vision, decreased far vision, myopia, miosis, ocular blurring, ocular discomfort, eye pain, decreased distance vision, and light sensitivity. According to the present invention, improvement of at least one parameter of vison selected from the group consisting of visual acuity, contrast sensitivity and stereopsis occurs in the substantial absence of miosis or myopia.

In certain embodiments of this aspect, the miotic drug is selected from carbachol, pilocarpine, physostigmine, and cevimeline or a pharmaceutically acceptable salt thereof. In certain embodiments, the methylated xanthine is selected from caffeine, theophylline, dyphylline, theobromine, aminophylline, and pentoxifylline and pharmaceutically acceptable salts thereof.

In certain embodiments, the at least one parameter of vision that is improved is selected from increased accommodation, increased far vision, increased near vision, increased contrast sensitivity, increased stereopsis or a combination thereof.

In certain embodiments of this aspect of the disclosure, the composition comprises about 3 to 8 wt% pilocarpine, 1 to 3 wt% carbachol, 0.25 to 3 wt% physostigmine, or 4 to 15 wt% cevimeline. In certain embodiments, the composition comprises about 0.05 to 1 wt% caffeine or pentoxifylline.

In certain embodiments, the composition comprises about 0.05 to 1 wt% caffeine, theophylline or pentoxifylline and about 3 to 8 wt% pilocarpine.

In certain embodiments, the composition comprises about 0.05 to 1 wt% caffeine, theophylline or pentoxifylline and about 1 to 3 wt% carbachol.

In certain embodiments, the composition comprises about 0.05 to 1 wt% caffeine, theophylline or pentoxiphylline and about 0.25 to 1 wt% physostigmine.

In another aspect of the disclosure, there is provided a topical composition for the treatment of astigmatism, presbyopia, hyperopia, decreased contrast sensitivity, or decreased stereopsis, said composition comprising a methylated xanthine and an ophthalmic miotic agent, wherein application of said composition to an outer surface of at least one eyelid of a subject in need thereof results in improvement of at least one parameter of vision affected by the astigmatism, presbyopia, hyperopia, or decreased contrast sensitivity, respectively, without inducing substantial miosis or myopia. In certain embodiments, the methylated xanthine is selected from caffeine, theophylline, dyphylline, theobromine, aminophylline, and pentoxifylline and pharmaceutically acceptable salts thereof. In certain embodiments, the ophthalmic miotic agent is pilocarpine, carbachol, physostigmine, or cevimeline.

### DETAILED DESCRIPTION OF THE DISCLOSURE

### Definitions

Unless defined otherwise, all technical and scientific terms used herein have the same meaning as commonly understood by one of ordinary skill in the art to which the subject matter pertains.

As used herein, the singular forms "a," "an" and "the" include plural referents unless the context clearly dictates otherwise.

The term "visual acuity" refers to sharpness or acuteness of vision, which is dependent on the sharpness of the retinal focus within the eye and is measured by the ability to discern letters or numbers at a given distance according to a fixed standard.

"Impaired visual acuity" as used herein means a decreased ability to see without corrective glasses or contact lenses. "Visual impairment" or "impaired visual acuity" is a best corrected visual acuity of worse than either 20/40 or 20/60. As used herein, the term "impaired visual acuity" does not refer to blindness or legal blindness, the latter of which is a visual acuity of 20/200 or worse.

The term "therapeutically effective amount" refers to an amount that is effective, when administered to an individual to treat presbyopia, hyperopia, astigmatism, decreased contrast sensitivity or decreased stereopsis as appropriate or improve at least one vision parameter. The extent of the vision improvement and/or success in the treatment of presbyopia, hyperopia, astigmatism, decreased contrast sensitivity or decreased stereopsis when a therapeutically effective amount of a composition is administered to an individual for the respective condition would be readily identifiable to a skilled person as is described herein.

The term "myopia" refers to a condition of the eye in which the visual images come to a focus in front of the retina resulting especially in defective vision of distant objects, causing nearsightedness.

The term "presbyopia" as used herein refers to a visual condition in which loss of elasticity of the lens of the eye causes defective accommodation and inability to focus sharply for near vision. Presbyopia is progressive age-related loss of ability to refocus from distance to near, resulting in loss of functional intermediate and near reading function. Presbyopia generally becomes apparent especially in middle age.

The term "hyperopia" or "farsightedness" as used herein refers to a condition of the eye where incoming rays of light impinge on the retina before converging into a focused image, resulting in difficulty seeing nearby objects clearly. The primary difference between presbyopia and hyperopia is what causes each condition. Age leads to presbyopia, whereas hyperopia has a genetic element. Hyperopia is present at birth and visual compensatory mechanisms worsen with age, resulting in blurry vision. Age often has no control over hyperopia, while presbyopia emerges in most people after age 40.

The term "astigmatism" as used herein refers to a visual defect in which the unequal curvature of one or more refractive surfaces of the eye, usually the cornea, prevents light rays from focusing clearly at a single point on the retina, resulting in blurred vision.

The term "contrast sensitivity" as used herein is the ability to detect subtle differences in shading and patterns. Contrast sensitivity is important in detecting objects without clear outlines and discriminating objects or details from their background. Contrast sensitivity is a measure of how much a pattern must vary in contrast to be seen while "visual acuity" measures how big an object must be to be seen. Contrast sensitivity is increasingly recognized as an important factor influencing the quality of vision. Among normally sighted people, both visual acuity and contrast sensitivity have a wide range of variation. In visual acuity, 0.8 (20/25, 6/9) is a low normal value; the highest normal values are three times higher, 2.5 (20/8, 6/2.5). Norms for different levels of contrast loss include profound loss at less than 0.48 log contrast, severe at 0.52-1.00, moderate 1.04-1.48, normal for individuals older than 60 years is 1.52-1.76 and normal for individuals age 60 and younger is 1.72-1.92 log contrast.

"Reduced contrast sensitivity" or "poor contrast sensitivity" as used herein means less contrast sensitivity as compared to a subject with "normal vision." Contrast sensitivity can be measured by the Pelli-Robson contrast chart, for example, which is used for most clinical research studies. The Pelli-Robson chart presents letters as triplets. Each triplet fades by 0.08 log units. Norms for different levels of contrast loss are "Normal" when the patient can read 6-7 lines (12-14 triplets). Moderate contrast loss is based on the patient only being able to read 4-5 lines. In severe loss, the patient can only read 2-3 lines; and with profound contrast loss, the patient can only read 1 line or less.

An alternative test to the Pelli-Robson chart is the Mars Letter Contrast Sensitivity Test. The Mars Test uses a set of 3 near charts. Each letter fades by 0.04 log units, allowing for a more precise measurement of contrast loss than is possible with the Pelli-Robson chart. Based on how many letters the patient is able to see, they are given a log contrast score. Unlike the Pelli-Robson test, which uses a 1-meter testing distance, the Mars test uses a 50-centimeter testing distance. Norms for different levels of contrast loss include profound loss at less than 0.48 log contrast, severe at 0.52-1.00, moderate 1.04-1.48, normal for individuals older than 60 years is 1.52-1.76 and normal for individuals age 60 and younger is 1.72-1.92 log contrast.

The term "sustained release" is used herein in its ordinary sense to mean that the compositions disclosed herein are designed to release a drug slowly over an extended period of time.

The term "uncorrected near visual acuity" ("UNVA") refers to a person's ability, without any vision aid (such as eyeglasses or contact lenses), to see the details of objects within arm's distance from the body (e.g., at 33-41 cm away from the eye). Similarly, the term "distance corrected near visual acuity" ("DCNVA") is used herein to refer to a person's ability to see the details of objects within arm's distance from the body (e.g., at 33-41 cm away from the eye), with the use of vision aids such as eyeglasses or contact lenses that correct for distance vision issues. The terms "near visual acuity," "near vision acuity," and "near vision" may be used interchangeably.

The term "uncorrected distance visual acuity" ("UDVA") refers to a person's ability, without any vision aid (such as eyeglasses or contact lenses), to see the details of objects beyond arm's distance from the body, e.g., greater than 4 meters away from the eye. The terms "distance visual acuity," "distance vision acuity," and "distance vision" may be used interchangeably.

The term "Comfort Score" as used herein refers to a score that rates pain sensitivity on a 0-10 scale, with a higher score associated with greater pain sensitivity. Comfort score ranges from 0 (poor comfort) through 4 (very comfortable). Comfort Score was assessed to measure comfort level which is known in the art to decline precipitously with standard cholinergic agonists due to inevitable accommodative spasm which causes pain. The discomfort and pain, due to ciliary muscle spasm and miosis cause eye pain, periocular pain, headache, stinging, burning and/or redness usually within 30 minutes of application of a miotic drug to the eye.

The term "Visual Clarity Score" refers to a subject's impression of the general quality of their vision. Clarity score ranges from zero (poor) to 4 (excellent). The clarity of overall vision in the current art with cholinergic therapy causes distance vision to become blurred with ciliary muscle spasm resulting in myopic near vision that is at a fixed distance. As a result, overall clarity of the visual experience diminishes.

The term "refraction" as used herein refers to a measurement of the focusing power of the eye at distance. Refraction refers to the bending of light that takes place within the human eye. Refractive errors include nearsightedness (myopia), farsightedness (hyperopia), and astigmatism.

The terms "intermediate vision", "intermediate vision acuity", and "intermediate visual acuity" may be used to refer to a person's ability to see the details of objects at distances between the near and far visual ranges. Such a distance range is considered to be a distance between approximately farther than arm's distance (about 40 to 60 cm away from the eye) and less than approximately 4 meters from the eye. The term distance-corrected intermediate visual acuity ("DCIVA") may be used to refer to a person's ability to see the details of objects at intermediate distances with the use of vision aids such as eyeglasses or contact lenses that correct for distance vision issues.

The term "vision parameter" or "parameter of vision" refers to any characteristic of a patient's vision that can be measured and is susceptible to being improved by the compositions and methods described herein. Vision parameters that may be improved in the various embodiments described herein include but are not limited to near vision acuity, intermediate visual acuity, distance visual acuity, night vision, day vision, optical aberrations (e.g., glare, light scattering), contrast sensitivity, stereopsis, and accommodative insufficiency. Vision or visual improvement, including but not limited to near, intermediate, and/or distance visual acuity, contrast sensitivity, stereopsis, and combinations thereof may for example be reflected in the increase of number of letters on a vision test chart correctly read at any time point post dosing, from baseline (i.e., from pre-treatment). Night vision improvement may be reflected in visual improvement for patients in dim or dark lighting (e.g., under mesopic or scotopic conditions). Day vision improvement may be reflected in visual improvement for patients in bright lighting as found during daylight hours or in sunshine (e.g., under photopic conditions). Improved stereopsis can provide improved 3-dimensional vision or depth perception. Vision improvement using the compositions and methods described herein can also be achieved in combination with or when using other visual aids and devices, including but not limited to reading glasses, lens modifying medications, and surgical presbyopic options including intraocular lenses (IOLs).

The term "accommodation" as used herein refers to the mechanism by which the eye changes focus from distant to near images. Accommodation is the adjustment of the optics of the eye to keep an object in focus on the retina as its distance from the eye varies. Accommodation is produced by a change in lens shape resulting from the action of the ciliary muscle on the zonular fibers. The lens is most malleable during childhood and the young adult years, progressively losing its ability to change shape with age, resulting in presbyopia.

The term "miosis" is used herein to mean excessive constriction of the pupil. Miosis can result from a normal response to an increase in light or can be caused by certain drugs such as miotics, e.g., cholinergic agents or pathological conditions.

The term "miotic drug" or "miotic(s)" or "ophthalmic miotic agent" or "ophthalmic miotic(s)" as used interchangeably herein means any drug or active pharmaceutical agent (API) that is used to treat a vision condition and which constricts the pupil of the eye when applied directly to the eyeball. Miotics are either parasympathomimetic (cholinergic-stimulating) drugs which have a direct muscarinic action, such as pilocarpine, cevimeline, and carbachol, or anticholinesterase drugs which block the effect of acetylcholinesterase thus letting acetylcholine produce its effect, such as physostigmine, neostigmine, echothiophate and demecarium. Some miotics act by blocking α-or β-adrenergic receptors. For example, dapiprazole and thymoxamine block the α-adrenergic receptors and propranolol blocks the β-adrenergic receptors. When applied directly to the eyeball, ophthalmic miotic agents often cause undesirable and painful side effects such as accommodative spasm, eye pain, headache, blurred vision, and loss of ability to function in less bright or dark ambient light.

As used herein the term "cholinergic agent" or "cholinergic agonist" refers to compounds which enhance or mimic the action of the neurotransmitter acetylcholine, the primary transmitter of nerve impulses within the parasympathetic nervous system, or butyrylcholine. A substance is cholinergic if it is capable of producing, altering, or releasing acetylcholine, or butyrylcholine ("indirect-acting"), or mimicking their behaviors at one or more of the body's acetylcholine receptor ("direct-acting") or butyrylcholine receptor types ("direct-acting"). Such mimics are called parasympathomimetic drugs or cholinomimetic drugs and are included in the category of "cholinergic agents" as used herein. The term "cholinergic agent" also refers to the salts and esters of such compounds. Non-limiting examples of cholinergic agents included in the compositions disclosed herein include the ophthalmic miotic agents, pilocarpine, carbachol, physostigmine, and cevimeline. In regard to pilocarpine, carbachol, physostigmine, and cevimeline, the terms "cholinergic agent," "cholinergic agonist," "miotic drug," "miotic(s)," "ophthalmic miotic agent," and "ophthalmic miotic(s)" are used interchangeably herein.

As used herein the term "methylated xanthine" or "methyl xanthine" refers to a compound included in a class of drugs that are derived from the purine base xanthine, which is produced naturally by both plants and animals. Non-limiting examples of methylxanthines include theophylline, dyphylline, caffeine, theobromine, aminophylline, and pentoxifylline.

The term "eyelid" as used herein means one of two movable folds of protective thin skin over each eye, with eyelashes and ciliary and meibomian glands along its margin. The upper and lower eyelids are separated by the palpebral fissure. It is understood that in each embodiment of the present methods for treating presbyopia, hyperopia, astigmatism, poor contrast sensitivity or decreased stereopsis, the therapeutic agent is applied to the outer skin surface of an upper, a lower, or both upper and lower eyelids of one or both eyes, as needed.

The inventors previously discovered that the addition of a methylated xanthine to a drug-containing topical composition for administration to the outer surface of an eyelid provides sustained release and enhanced penetration of the drug into the eye. (U.S. 9,034,830).

Without wishing to be tied to one particular theory, it is believed that the methylated xanthine, e.g., caffeine, can serve as a solubilizing agent for active agent(s) included in the composition, making the active agent(s) more bioavailable. It is also believed that the methylated xanthine acts on the blink muscle, causing small, local, involuntary muscle contractions (muscle twitches) that drive the drug contained within the composition into the eyelid and circulatory system of the eye.

The inventors have now discovered that application of a composition comprising a combination of a methylated xanthine and a cholinergic agent or other miotic drug to the eyelid(s) of an individual diagnosed with or having vision disorders causing blurred vision such as hyperopia, astigmatism, presbyopia, or poor contrast sensitivity or decreased stereopsis results in improved distance vision, peripheral vision, or near vision, or improved contrast sensitivity or stereopsis, depending on the condition being treated, without the adverse side effects associated with application of a miotic drug such as a cholinergic agent to the eye, e.g., via eye drops. The compositions and methods of the present disclosure treat presbyopia by improving depth of focus and producing accommodation without producing eye pain, headache, blurred vision, induced myopia, miosis, loss of distance vision, and/or loss of vision in dim or dark light conditions by the topical application of a composition of the disclosure to the eyelid or eyelids of a presbyopic subject. The compositions and methods of the disclosure treat hyperopia by improving near vision and treat astigmatism by improving visual clarity, for example. The present compositions and methods treat poor contrast sensitivity by enhancing contrast sensitivity. In the treatment of each of these eye conditions, the topical application of a composition of the disclosure comprising a combination of a methylated xanthine and a cholinergic agent or other miotic drug to the eyelid(s) results in improved vision without occurrence of miosis or myopia.

The compositions and methods of the present disclosure selectively impact a subject's focusing or accommodating ability without significantly impacting the pupil. The methods of the disclosure result in absorption of the inventive compositions into the ciliary body, which is a ring-shaped thickening of tissue inside the eye that divides the posterior chamber from the vitreous body. Absorption into the ciliary body is *via* the vascular system of the eyelid. As a result, the present compositions and methods do not result in a bolus of active ingredient(s) being pumped into the anterior chamber of the eye. The methods and compositions of the disclosure affect a subject's ability to focus without causing ciliary spasms and with little or no significant effect on the pupil. The methods and compositions of the disclosure are thus able to maintain the physiological range of pupillary size. That is, the aperture of the pupil remains in the physiological range of pupillary size (which depends on eye color) after application of a composition of the disclosure to the eyelid, thus avoiding loss of contrast sensitivity, or night blindness, or loss of visual acuity.

A fully dilated pupil is typically in the 4 to 8 millimeters in size, while a constricted pupil is under 3 mm in size, typically pinpoint pupil size, i.e., 1.5 to 1.9 mm, or as large as 2.5 mm. According to the American Academy of Ophthalmology, pupils generally range in size from 2 to 8 mm. The methods and compositions of the disclosure maintain the pupils within this range, while application of miotics alone to the eye cause the pupil to significantly constrict, e.g., below 2.5 mm.

In medicine, the use of cholinergic agonists and other miotics is limited because of their propensity to cause adverse effects in any organ under the control of the parasympathetic nervous system; adverse effects include blurred vision, miosis, myopia, cramps and diarrhea, low blood pressure and decreased heart rate, nausea and vomiting, salivation and sweating, shortness of breath, and increased urinary frequency. The current use of the cholinergic compound, pilocarpine in ophthalmic eyedrop solutions for example is limited by several commonly-experienced adverse events, including temporal and periorbital headache (i.e., brow ache), which can be due at least in part to rapid ciliary muscle contraction, ciliary spasms, reduction in night and dim light vision due to miosis, reduction in distance vision, reduction in contrast sensitivity and myopia. Pilocarpine also acts on the muscarinic cholinergic receptors found on the iris sphincter muscle, causing the muscle to contract, resulting in pupil constriction (i.e., miosis) (Levin et al., Adler's Physiology of the Eye, 11th edition by Saunders Elsevier (Edinburgh), pp. 56, 57, and 509-510). Due to these adverse effects, pilocarpine is only used at low doses, e.g., 1.0 - 2 % w/v in eyedrops and is only applied monocularly due to the loss of distance vision and myopia. Similarly, carbachol, a cholinomimetic compound, which is applied to the eye as a solution (eyedrops) to treat glaucoma, causes decreased night vision and is only applied at low dosages, i.e., about 1.0 %. Due to the low dosages required to limit adverse side effects, any beneficial effects of these drugs are of short duration, e.g., four hours or less, requiring several applications per day, which further exacerbates the adverse effects.

In order to combat the adverse side effects often observed with the use of pilocarpine eyedrops, the drug is often combined with a cholinergic antagonist. However, such combinations often implicate additional side effects on top of those related to use of pilocarpine.

Use of the compositions and methods of the disclosure eliminates or at least significantly reduces the adverse effects observed with the use of eyedrops containing cholinergic compounds or other miotic drugs such as pilocarpine, physostigmine or carbachol, e.g., miosis or myopia. Moreover, because the compositions of the disclosure are formulated for application to the eyelid(s) rather than directly to the eyeball, the cholinergic agent or other miotic drug contained therein is slowly released into and across the eyelid and into the eye, enabling the use of higher dosages of active ingredient and hence, longer lasting beneficial effect on vision.

In one aspect of the disclosure there are provided ophthalmic topical compositions for application to the eyelid for the treatment of presbyopia, astigmatism, hyperopia or poor or reduced contrast sensitivity or decreased stereopsis. In embodiments described herein, compositions containing a therapeutic amount of a cholinergic agent or other miotic drug in combination with an effective amount of a methylated xanthine is topically applied to one or more eyelids of a presbyopic or hyperopic subject, or a subject with astigmatism or subject with poor contrast sensitivity or poor stereopsis to treat the respective ophthalmic condition.

The compositions described herein are formulated for topical application to the eyelid(s) of a subject. The term "topical" as employed herein relates to the use of a composition, as described herein, incorporated in a suitable pharmaceutical carrier and applied to the outer surface of one or more eyelids. The composition can be applied to the outer surface of the eyelid(s) of one or both eyes, as needed. Typical topical compositions are pharmaceutically acceptable forms in which the composition is applied directly to the outer skin surface of the eyelid. Non-limiting examples of such pharmaceutical forms include ointments, liniments, creams, shampoos, lotions, pastes, jellies, sprays, aerosols. The compositions also can be applied via patches or impregnated dressings. The term "ointment" includes formulations having oleaginous, water-soluble and emulsion-type bases, e.g., petrolatum, lanolin, polyethylene glycols, as well as mixtures of these.

Methylated xanthines that can be used in the various compositions and methods of the disclosure include for example, caffeine, theophylline, dyphylline, or aminophylline topical application to the eyelid or eyelid margin.

Cholinergic agents useful for inclusion in the compositions described herein include but are not limited to pilocarpine, carbachol, physostigmine, cevimeline, neostigmine, an acetylcholinesterase inhibitor, donepezil, galanthamine, tacrine, rivastigmine, metrifonate, pyridostigmine, ambenonium, demarcarium, cevimeline, edrophonium, huperzine A, ladostigil, diisopropyl fluorophosphate (Floropryl), phospholine iodide (echothiophate) and physostigimine (eserine), and their pharmaceutically acceptable salts and esters. In certain embodiments of every aspect of the disclosure, the cholinergic agent is pilocarpine, physostigmine, carbachol, cevimeline, or a pharmaceutically acceptable salt or ester thereof, or a combination thereof.

In general, the compositions of the disclosure comprise one or more cholinergic or other miotic agents in therapeutically effective amounts of from about 0.1 to about 10 wt % of the composition. Because the compositions described herein are applied to the eyelid, rather than directly to the eyeball as in the case of eyedrops, higher amounts of the active agent, e.g., a cholinergic agent can be included in the compositions, thereby improving the beneficial effects on vision observed with use of these agents, without the usual side effects seen with their direct use in the eye. For example, application of an amount of 1 to 10 wt% or 3 to 8 wt% or 4 to 6 wt%, or 6 to 8 wt% of cholinergic agent such as pilocarpine, or an amount of from 1 to 3 wt% or 1.5 to 2.5 wt% or 2 to 3 wt% of carbachol or an amount of from 0.25 to 3 wt% or 0.5 to 2.5 wt% or 1 to 2 wt% physostigmine, or 4 to 15 wt%, 4 to 12 wt% or 4 to 10 wt% cevimeline, for example applied to the eyelid(s) improves visual clarity and near vision for example and/or contrast sensitivity and/or stereopsis in the absence of or at least a significant reduction in negative effects on distance vision, miosis, or myopia, which are observed with application to the eye of the miotic agent alone. Application of the disclosed compositions to the eyelid of a subject in need of treatment results in a lower incidence of at least one or more adverse events such as ocular blurring, ocular discomfort, eye pain, brow ache, blurry vision, light sensitivity, night blindness, pinhole vision, loss of contrast sensitivity, myopia and decreased far vision, compared to administration of an eyedrop solution comprising pilocarpine, carbachol, phsyiostigmine or other cholinergic agent. Further, application of the compositions described herein to the eyelid results in a sustained release of the cholinergic or miotic agent into the eye due to the inclusion of the methylated xanthine in the compositions which causes small, local involuntary muscle contractions (a muscle twitch) in the blink muscle for a sustained period of time, driving the active ingredient into the eye over a sustained period of time, e.g., at least 4 hours and up to at least 10 hours or at least 12 hours or up to 24 hours after application. In the embodiments described herein, compositions can be administered once daily, twice daily, or more often, as needed. In certain embodiments, the compositions are administered once daily. When administered, the compositions can have a duration of action sufficient for an entire day. In some embodiments, the compositions can have a duration of effect of at least four hours, such as more than 4 hours, such as at least 6 hours, at least 8 hours, at least 10 hours, at least 12 hours, or up to 24 hours, as well as all intervening time points. Some embodiments can provide for a composition having a duration of action 12 hours, or even 24 hours. The duration of action refers to the duration of time that the administered composition has a positive effect on at least one vision parameter associated with presbyopia, hyperopia, astigmatism, decreased contrast sensitivity or decreased stereopsis. In certain embodiments of all aspects of the disclosure, the compositions of the disclosure can include 1 to 10 wt%, e.g., 2 to 9, 3 to 9 wt%, 4 to 9 wt%, 5 to 9 wt%, or 6 to 9 wt% or any amount therein between, of any one of a cholinergic agent such as pilocarpine, physostigmine, cevimeline, or carbachol or their pharmaceutically acceptable salts or esters for example, in combination with 0.05 to 10 wt% methylated xanthine, such as caffeine, theophylline, pentoxifylline, aminophylline, or theobromine. Other examples of compositions of the disclosure include compositions containing 6 to 8 wt% pilocarpine, or 1 to 3 wt% carbachol or 0.25 to 3 wt% physostigmine, or 4 to 15 % cevimeline (or pharmaceutically acceptable salts or esters of these cholinergic agents) in combination with 0.05, 0.1, 0.2, 0.3, 0.4, or 0.5 wt% caffeine or other methylated xanthine. In some embodiments, the amount of methylated xanthine used with any of the cholinergic or miotic agents included in the compositions described herein is 0.05 to 2.5 wt%, such as 0.06, 0.07, 0.08, 0.09, 0.1, 0.2, 0.3, 0.4, 0.5, 0.6, 0.7, 0.8, 0.9, 1.0, 1.1, 1.2, .13, 1.4, 1.5, 1.6, 1.7, 1.8, 1.9, 2.0, 2.1, 2.2, 2.3, 2.4, or 2.5 wt%. In certain embodiments the amount of caffeine in the composition of the disclosure is 0.2 to 0.5 wt %, such as 0.32 wt%. In other embodiments, the amount of pentoxifylline is from 0.05 to 1 wt%. In some embodiments the amount of theophylline included in a composition disclosed herein is 0.1 to 0.5 wt%.

Compositions of the disclosure include, but are not limited to, compositions containing 1 to 10 wt% pilocarpine and 0.05 to 1 wt% caffeine; 1 to 10 wt% pilocarpine and 0.05 to 1 wt% theophylline; 1 to 10 wt% pilocarpine and 0.05 to 1 wt% pentoxifylline; 1 to 3 wt% carbachol and 0.05 to 1 wt% caffeine; 1 to 3 wt% carbachol and 0.05 to 1 wt% theophylline; 1 to 3 wt% carbachol and 0.05 to 1 wt% pentoxifylline; 0.25 to 3 wt% physostigmine and 0.05 to 1 wt% caffeine; 0.25 to 3 wt% physostigmine and 0.05 to 1 wt% pentoxifylline; 0.25 to 3 wt% physostigmine and 0.05 to 1 wt% theophylline; 4 to 15 % cevimeline and 0.05 to 1 wt% caffeine; 4 to 15 wt% cevimeline and 0.05 to 1 wt% theophylline; 4 to 15 wt% cevimeline and 0.05 to 1 wt% pentoxifylline.

The compositions described herein can be formulated in an aqueous solution, cream, ointment or physiologically acceptable oil. Such formulations may or may not, depending on the dispenser, contain preservatives such as benzalkonium chloride, chlorhexidine, chlorobutanol, parahydroxybenzoic acids and phenylmercuric salts such as nitrate, chloride, acetate, and borate, or antioxidants, as well as additives like EDTA, sorbitol, boric acid, and the like as additives. Furthermore, aqueous solutions may contain viscosity increasing agents such as polysaccharides, e.g., methylcellulose, mucopolysaccharides, e.g., hyaluronic acid and chondroitin sulfate, or polyalcohol, e.g., polyvinylalcohol. For ease in application, the composition can be formulated as a stick applicator or other cosmetic applicator appropriate for application to the eyelids or base of the eyelashes, e.g., at the eyelid margin.

The disclosure also provides for the use of a methylated xanthine in combination with an ophthalmic miotic agent as described herein, in the manufacture of a medicament for treating a subject experiencing decreased visual acuity, decreased contrast sensitivity or decreased stereopsis.

Kits containing one or more compositions disclosed herein are also provided. The kits may include single use dosage forms of composition disclosed herein or may include a vial for example containing multiple doses that are to be metered out by the patient or via the applicator itself. Further, kits may include formulations of different therapeutic agents for treatment of presbyopia, intended to be applied to different eyelids or areas of the skin and/or at different times during the treatment regimen. Kits will also include, when necessary, instructions for the appropriate dosing regimen.

The following examples are illustrative in nature and are in no way intended to be limiting.

### EXAMPLES

### Example 1. Immediate effect of treatment.

A 45 year old patient who had recently noticed difficulty with her close up vision was treated with a topical composition containing 6 wt% pilocarpine and 0.32 wt% caffeine. The composition was applied to the eyelids of both eyes.

Prior to treatment the following data points were obtained:
Pupil
Refraction: +25SPH
Plano SPH > +1.75 add
Uncorrected Near
Uncorrected DV
Comfort (1+ to 4+)
Clarity (1+ to 4+)
Pre-treatment pupil 5.0mm
Near vision JS
Distance vision sc 20/20
Comfort 3+
Clarity 3+

The following post-treatment measurements were made:
Hour 1 pos- treatment:
   4.5mm pupil,
   Uncorrected Near vision (VA) increased to J2,
   Uncorrected Distance vision remained 20/20,
   Comfort Score improved to 4+,
   Clarity Score improved to 4+
Hour 2 post treatment
   Pupil size 5.0mm
   Uncorrected Near vision increased to the highest level at J1
   Uncorrected Distance VA 20/20,
   Comfort Score 4+
   Clarity Score 4+
Hour 3 post-treatment
   Pupil Size 4.5mm
   Near vision remained increased at J1
   Distance vision stayed 20/20,
   Comfort Score 4+
   Clarity Score 4+
Hour 4 post-treatment
   Pupil size 4.5mm
   Near vision remained increased at J1
   Distance vision remained 20/20,
   Comfort Score 4+
   Clarity Score 4+
Hour 5 post-treatment
   Pupil size 4.0
   Near vision remained optimal at J1
   Distance vision remained perfect at 20/20,
   Comfort Score 4+
   Clarity Score 4+

The increase in near vision was sustained for 12 hours after the single treatment. No adverse effects were observed.

Treatment Summary: Emmetropic presbyopia treated with pilocarpine 6% and 0.32% caffeine resulted in ability to generate voluntary near and intermediate distance vision with preservation of distance vision in each eye. Distance vision was improved in the right eye.

### Example 2.

A 49 year old female presented with hyperopic astigmatism with precipitous onset of presbyopia and loss of near and distance vision in both eyes. The patient, due to intolerance, was not a candidate for contact lenses. Additionally, the patient experienced asthenopia and constricted visual field with nausea when wearing glasses.

Prior to treatment the patient presented with the following:
Pretreatment:
   Refraction:
   OD: +1.25 +1.25 x 105. 20/20, +2.00 add
   OS: +1.75 +0.25 x 105 20/20, +2.00 add
Pretreatment:
   Uncorrected Vision:
   Distance: 20/80 Right Eye, 20/30 Left Eye
   Near: Unable to read reading card at all.
   Pupils: 5mm Right Eye (OD), 4.8mm Left Eye (OS)
   Comfort Score 2+
   Clarity Score zero

The patient was treated with a topical composition comprising 0.32 wt% caffeine and 6 wt% pilocarpine. The composition was applied to the outer surface of both eyelids.

The composition was applied to the eyelids once daily in the morning for three days. The patient reported that her Near vision improved immediately after the first treatment and the improvement lasted throughout the day/evening after a single application of the composition in the morning.

After three days of a once daily application of the composition the following data were obtained:
Post Treatment
Uncorrected vision:
Distance increased to 20/25 Right Eye, 20/20 Left Eye
Near vision: J4, able to read her phone and texts now Easily
Pupils: 5mm Right Eye, 4.5mm Left Eye
Comfort increased to 4+, Clarity 4+

With ongoing treatment, the patient's reading vision increased to J2 uncorrected reading vision and she maintained excellent improved distance vision in each eye. The patient was able to forego wearing glasses for both distance and near vision while undergoing once daily treatment for a year.

Treatment Summary: Hyperopia with astigmatism with baseline poor vision and severe presbyopia (with blurred near and intermediate vision) treated with pilocarpine 6% and 0.32% caffeine resulted in marked improvement of distance vision in each eye. Voluntary reading ability at near and intermediate vision was restored.

### Example 3.

A 54 year old male subject who had recently noticed difficulty with close up vision was treated with a topical composition containing 8 wt% pilocarpine and 0.10 wt% theophylline. The composition was applied to the eyelids of both eyes.

Prior to treatment the following data points were obtained:
Pupil 5.0mm

Refraction:
OD +0.25+0.50 @ 015. +2.00 reading Add
OS +0.25 +0.25 @ 175. +2.00 reading Add

### Pre-treatment

Near vision uncorrected J10, which is 20/100 at near,
Uncorrected distance vision 20/25 right eye, 20/20 left eye
Comfort Score2+
Clarity Score1+

The following post-treatment measurements were made:
Post Treatment: Hour 1 post- treatment: 4.5mm pupil; Near vision increased to J8; Distance vision increased to 20/20 in each eye.
Comfort Score 3+
Clarity Score 1+
Hour 2 post-treatment: Pupil size 5.0mm, Near vision increased again J7; Distance vision 20/20 Right eye and increased to 20/15 left eye
Comfort Score 4+
Clarity Score 2+
Hour 3 post-treatment: Pupil Size 4.5mm, Near vision remained increased to J5; Distance vision stayed 20/20 right eye and 20/15 left eye
Comfort Score 4+
Clarity Score 2+
Hour 4 post-treatment: Pupil size 4.5mm, Near vision remained increased to J4; Distance vision remained 20/20 right eye and 20/15 left eye each eye
Comfort Score 4+
Clarity Score 4+
Hour 5: Pupil size 4.0, Near vision optimal at J1; Distance vision remained at 20/20 each eye,
Comfort Score 4+
Clarity Score 4+

The increase in Near Vision was sustained for 12 hours after the single treatment. No adverse effects were observed.

### Example 4.

A 56 year old subject losing his close up vision was treated with a topical composition containing 3 wt % Carbachol and 0.16 wt% Theophylline. The composition was applied to the eyelids of both eyes.

Prior to treatment the following data points were obtained:
Pre-treatment:
   Pupil 5.0mm
   Near vision uncorrected J7 which is 20/100 at near
   Uncorrected distance vision 20/20 in each eye
   Comfort Score 2+
   Clarity Score 1+
Refraction:
   OD: -0.25 sph. Add +2.00
   OS: -0.75 sph. Add +2.00
Hourly measurements were checked post- treatment:
   Hour 1 post treatment: 5.0mm pupil; Near vision increased to J5; Distance vision remained 20/20 each eye
      Comfort Score 3+
      Clarity Score 1+
   Hour 2 post-treatment: Pupil size 4.5mm; Near vision increased again J2; Distance vision 20/20 in each eye
      Comfort Score 4+
      Clarity Score 4+
   Hour 3 post-treatment: Pupil Size 4.5mm; Near vision increased again to J1; Distance vision stayed 20/20 in each eye
      Comfort Score 4+
      Clarity Score 4+
   Hour 4 post-treatment: Pupil size 4.0mm; Near vision remained high at J1; Distance vision remained 20/20 each eye
      Comfort Score 4+
      Clarity Score 4+
   Hour 5 post-treatment: Pupil size 5.0; Near vision now optimal at J1; Distance vision remained at 20/20 in each eye
      Comfort Score 4+
      Clarity Score 4+

The increase in near vision was sustained for 12 hours after the single treatment. No adverse effects were observed.

### Example 5.

Pentoxifylline with two direct acting cholinergic agonist API's: Pilocarpine and Carbachol
Pentoxifylline 0.05% with Pilocarpine 8%
Pentoxifylline 1.0 % with Pilocarpine 8%
Pentoxifylline 0.05% with Carbachol 3%
Pentoxifylline 1.0% with Carbachol 3%

### PENTOXIFYLLINE DOSE RANGE STUDY:

### LOW DOSE PENTOXIFYLLINE 0.05%: Hyperopia and Presbyopia treated with 0.05% pentoxifylline and 8% Pilocarpine

A 59 year old patient with poor close up vision and blurry distance vision was treated with a topical composition containing 8 wt% pilocarpine and 0.05 wt% pentoxifylline. The composition was applied to the eyelids of both eyes.

Prior to treatment the following data points were obtained:
Pre-treatment pupil 4.0mm,
Near vision uncorrected J8, which is 20/80 at near,
Uncorrected distance vision 20/40 OD, 20/25 OS
Comfort Score 2+
Clarity Score 1+
OD: +1.00 Sphere with +2.00 ADD for reading
OS: +1.75 Sphere with +2.00 ADD for reading

The following post-treatment measurements were made:
Hour 1 post treatment:
   Pupil size 4.5mm
   Near vision increased to J6
   Distance vision remained 20/40 OD, 20/25 OS
   Comfort Score 3+
   Clarity Score 2+
Hour 2 post-treatment:
   Pupil size 4.0mm
   Near vision increased to a more functional level J4, which is 20/40
   Distance vision improved to 20/20 in each eye.
   Comfort Score 4+
   Clarity Score 4+
Hour 3 post-treatment:
   Pupil Size 3.5mm
   Near vision remained increased at J4
   Distance vision stayed at an improved level of 20/20 in each eye
   Comfort Score 4+
   Clarity Score 4+
Hour 4 post-treatment:
   Pupil size 3.5 mm
   Near vision jumped again to J2
   Distance vision remained increased to 20/20 in each eye
   Comfort Score 4+
   Clarity Score 4+
Hour 5 post-treatment:
   Pupil size 3.5
   Near vision now achieved optimal level of acuity at J1
   Distance vision remained excellent at 20/20,
   Comfort Score 4+
   Clarity Score 4+

The increase in near vision was sustained for 12 hours after the single treatment. No adverse effects were observed.

### HIGH DOSE PENTOXPHYLLINE 1.0 %: Hyperopia and Presbyopia treated with 1.0% pentoxifylline and 8% Pilocarpine

A 59 year old subject with poor close up vision and blurry distance vision was treated with a topical composition containing 8 wt% pilocarpine and 1.0 wt% pentoxifylline. The composition was applied to the eyelids of both eyes.

Prior to treatment the following data points were obtained:
Pre-treatment pupil 4.0 mm,
Near vision uncorrected J8, which is 20/80 at near,
Uncorrected distance vision 20/40 OD, 20/25 OS
Comfort Score 2+
Clarity Score 1+
OD: +1.00 Sphere with +2.00 ADD for reading
OS: +1.75 Sphere with +2.00 ADD for reading

The following post-treatment measurements were made:
Hour 1 post-treatment:
   Pupil size 3.5mm
   Near vision increased to J4
   Distance vision improved to 20/25 OD, 20/20 OS
   Comfort Score 4+
   Clarity Score 3+
Hour 2 post-treatment:
   Pupil size 3.0 mm
   Near vision increased to a near optimal level J2, which is 20/25
   Distance vision improved to a crisp 20/20 in each eye.
   Comfort Score 4+
   Clarity Score 4+
Hour 3 post-treatment:
   Pupil Size 3.5mm
   Near vision optimized to J1, which is 20/20
   Distance vision remained optimal at 20/20 in each eye
   Comfort Score 4+
   Clarity Score 4+
Hour 4 post-treatment:
   Pupil size 3.0 mm
   Near vision stable at J1, which is 20/20
   Distance vision remained increased to 20/20 in each eye
   Comfort Score 4+
   Clarity Score 4+
Hour 5 post-treatment:
   Pupil size 3.5
   Near vision stable at optimal level of acuity at J1
   Distance vision remained excellent at 20/20,
   Comfort Score 4+
   Clarity Score 4+

The increase in near vision was sustained for 12 hours after the single treatment. No adverse effects were observed.

### Example 6.

### PENTOXIFYLLINE DOSE RANGE WITH CARBACHOL 3%: Methyl Xanthine Low Dose Range: Pentoxifylline 0.05% with Carbachol 3%

A 54 year old female presented with mild hyperopic astigmatism with precipitous onset of presbyopia and loss of near and distance vision in both eyes. The patient, intolerant to contact lens training and presenting with anxiety, was not a candidate for contact lenses or ophthalmic surgery. Additionally, the patient experienced chronic asthenopia with nausea when wearing glasses.

Prior to treatment the patient presented with the following:
Pre-treatment:
   Refraction:
   OD: +0.25 +0.50 x 105 20/20, +2.00 add
   OS: +0.25 +0.25 x 105 20/20, +2.00 add
Uncorrected Vision:
   Distance: 20/30+3 Right Eye, 20/20-3 Left Eye
   Near: Unable to read reading card at all.
   Pupils: 5mm both eyes
   Comfort Score zero
   Clarity Score zero

The patient was treated with a topical composition comprising 0.05 wt% Pentoxifylline and 3 wt% Carbachol. The composition was applied to the outer surface of both eyelids.

The composition was applied to the eyelids once daily in the morning for three days. The patient reported that her near vision improved immediately after the first treatment and the improvement lasted throughout the day/evening after a single application of the composition in the morning.

After three days of a once daily application of the composition the following data were obtained:
Uncorrected vision:
Distance increased to 20/25 Right Eye, and crisper at 20/20 Left Eye
Near vision: J4, functionally able to read the phone and texts now
Pupils: 4.5mm Both Eyes
Comfort core increased to 2+
Clarity Score 3+

The patient was able to forego wearing glasses for both distance and near vision while undergoing once daily treatment.

### Example 7.

### Methyl Xanthine Pentoxifylline High Dose Range: Pentoxifylline 1% with Carbachol 3%

The 54 year old female patient described in example 6 stopped treatment for four weeks in order to "wash out" the effects of 0.5 wt% pentoxifylline and 3 wt% carbachol. After four weeks the patient was treated with a higher dose of pentoxifylline (1 wt%) and 3 wt% carbachol.

Prior to treatment with 1 wt% pentoxifylline and 3 wt% carbachol, the patient presented with the following:
Refraction:
   OD: +0.25 +0.50 x 105 20/20, +2.00 add
   OS: +0.25 +0.25 x 105 20/20, +2.00 add
Uncorrected Vision:
   Distance vision: 20/30+3 Right Eye, 20/20-3 Left Eye
   Near vision: Unable to read reading card at all.
   Pupils: 5mm both eyes
   Comfort Score zero
   Clarity Score zero

The subject was treated with a topical composition comprising 1.0 wt% Pentoxifylline and 3 wt% Carbachol. The composition was applied to the outer surface of both eyelids.

The composition was applied to the eyelids once daily in the morning for three days. The subject reported that her near vision improved immediately after the first treatment and the improvement lasted throughout the day/evening after a single application of the composition in the morning.

After three days of a once daily application of the composition the following data were obtained:
Uncorrected vision:
Distance increased to 20/25 Right Eye, and crisper at 20/20 Left Eye
Near vision: J1, now easily able to read menus, phone, and computer w/ 20/20 VA
Pupils: 4.0 mm Both Eyes
Comfort Score increased to 4+
Clarity Score 4+

The subject was able to forego wearing glasses for both distance and near vision while undergoing once daily treatment with notable ease of reading (described by the subject as "really sharp").

### Example 8.

### 1 wt% Physostigmine and 0.32 wt% Caffeine

A 54 year old patient who had recently noticed difficulty with close up vision was treated with topical application to the outer eyelids of both eyes with a composition containing 1 wt% physostigmine and 0.32 wt% caffeine.

Prior to treatment the following data were obtained:
Refraction:
OD +0.25+0.50 @ 015. +2.00 reading Add
OS +0.25 +0.25 @ 175. +2.00 reading Add
Pre-treatment pupil 5.0mm,
Near vision uncorrected J10, which is 20/100 at near,
Uncorrected distance vision 20/25 Right eye, 20/20 left eye
Comfort 2+, clarity 1+

The following post-treatment measurements were made:
Hour 1 post treatment 4.5mm pupil, Near vision increased to J9, Distance vision increased to 20/20 each eye
   Comfort 3+, Clarity 1+
Hour 2, Pupil size 5.0mm, Near vision increased again J7, Distance vision 20/20 Right eye and increased to 20/15 left eye
   Comfort 4+, Clarity 2+
Hour 3, Pupil Size 4.5mm, Near vision increased to J5, Distance vision stayed 20/20 right eye and 20/15 left eye
   Comfort 4+, Clarity 2+
Hour 4, Pupil size 4.5mm, Near vision increased again to J3 , Distance vision remained 20/20 right eye and 20/15 left eye each eye,
   Comfort 4+, Clarity 4+
Hour 5, Pupil size 4.0, Near vision now optimal at J1, Distance vision remained at 20/20 each eye,
   Comfort 4+, Clarity 4+

The increase in Near vision was sustained for 12 hours after the single treatment. No adverse effects were observed.

### Example 9.

Three subjects were treated with a composition containing 6 wt% pilocarpine and 0.32 wt% caffeine. A 46 year old male, a 42 year old male, and a 53 year old male all presented with decline in reading vision requiring reading glasses. All were distance sighted emmetropes.

**Subject 1:** 46 year old male
Baseline Distance vision 20/25, CS of 50% (LogCS 0.3)
Baseline Near Vision. 20/114, Snellen Jaeger 11, CS 40%
Baseline Stereopsis: Zero stereo vision

The subject was treated by application of a lotion containing 6 wt% Pilocarpine and 0.32 wt% caffeine to the outer skin surface of the eyelids of both eyes. Visual improvement and contrast sensitivity improvement were seen within three hours of treatment and remained optimal for 12 hours.

Near Vision: Baseline vision of Jaeger 11, Snellen 20/114 at near with 39.81% contrast at near (log CS 0.4). Within 2 hours of binocular treatment the vision increased significanty to 20/25, Jaeger 2 at near with 15.85% CS (log 0.8).

Near Vision Contrast sensitivity more than doubled at 260% (2.6 times) better than baseline.

Distance Vision: Baseline vision of 20/25, Contrast sensitivity of 50.12% (log 0.3). Distancer vision increased with treatment to 20/20 with contrast sensitivity of 39.81% (log 0.4).

Distance Vision contrast sensitivity improved to 33% better than baseline

Stereopsis: Binocular vision improved to 8/9 circles which is a near perfect score.

Subject 2: 42 year old male
Baseline Distance Vision: 20/20, CS 39.81% (log 0.4)
Baseline Near Vision: Snellen 20/40, Jaeger 4, CS 50.12% (log 0.3)
Baseline Stereopsis: 5/5 circles

The subject was treated by application of a lotion containing 6 wt% Pilocarpine and 0.32 wt% caffeine to the outer skin surface of the eyelids of both eyes. Visual improvement and contrast sensitivity improvement were seen within three hours of treatment and remained optimal for 12 hours.

Near Vision: Baseline Near Vision: Snellen 20/40, Jaeger 4, CS 50.12% (log 0.3). Within 2 hours of binocular treatment, the subject's vision increased to 20/20, Jaeger 1 at near with 15.85% CS (log 0.8).

Near Vision Contrast sensitivity more than tripled at 333% ( 3.3 times) better than baseline.

Distance Vision: Baseline vision of 20/20, Contrast sensitivity of 39.81%(log 0.4). Distance vision increased with treatment to 20/20 with contrast sensitivity of 19.95% (log 0.7). Distance Vision contrast sensitivity doubled, which is 100% better than baseline.

Stereopsis: Binocular vision measurement improved significantly, doubling from baseline to a perfect score of 9/9 circles.

Subject 3: 53 year old male
Baseline Distance Vision: 20/25, CS 50.12 % (log 0.3)
Baseline Near Vision: Snellen 20/114, Jaeger 11, CS 79.43%(log 0.1)
Stereopsis: Failed Titmus Test, Zero Depth Perception

The subject was treated by application of a lotion containing 6 wt% Pilocarpine and 0.32 wt% caffeine to the outer skin surface of the eyelids of both eyes. Visual improvement and contrast sensitivity improvement were seen within three hours of treatment and remained optimal for 12 hours.

Near Vision: Baseline Near Vision: Snellen 20/114, Jaeger 11, CS 50.12% (log 0.3). Within 6 hours of binocular treatment, the subject's vision increased significantly to 20/30, Jaeger 3 at near with 25.12% CS (log 0.6).

Near Vision Contrast sensitivity doubled at 100% (2 times) better than the poor baseline contrast sensitivity at low visual acuity.

Distance Vision: Baseline vision of 20/25, Contrast sensitivity of 50.12% (log 0.3). Distance vision increased with treatment to 20/20 with contrast sensitivity of 25.12% (log 0.6).

Distance Vision contrast sensitivity doubled, which is 100% (2 times) better than baseline.

Stereopsis: Binocular vision improved from a baseline failure to a near perfect score of 8/8 circles.

## Claims

1. An ophthalmic miotic agent for use in treating impaired visual acuity and lowering the incidence of at least one adverse effect associated with application of the miotic agent to the eye of a subject, in combination with at least one methylated xanthine or pharmaceutically acceptable salt or ester thereof, wherein said combination is formulated for topical application and applied to at least one eyelid of the subject; and wherein the at least one adverse effect is selected from miosis and myopia.

2. The ophthalmic miotic agent for the use of claim 1, wherein the ophthalmic miotic agent comprises pilocarpine, carbachol, cevimeline or physostigmine.

3. The ophthalmic miotic agent for the use of claim 1, wherein the combination comprises 1 to 10 wt% pilocarpine, 1 to 3 wt% carbachol, 4 to 15 wt% cevimeline, or 0.25 to 3 wt% physostigmine.

4. The ophthalmic miotic agent for the use of any one of claims 1 to 3, wherein the methylated xanthine is selected from caffeine, theophylline, dyphylline, theobromine, aminophylline, and pentoxifylline and pharmaceutically acceptable salts thereof.

5. The ophthalmic miotic agent for the use of claim 4, wherein the methylated xanthine comprises 0.05 to 10 wt% of the combination.

6. The ophthalmic miotic agent for the use of any one of claims 1 to 5, wherein the combination results in improvement of at least one parameter of visual acuity.

7. The ophthalmic miotic agent for the use of claim 6, wherein the at least one parameter of visual acuity is improved for at least four hours.

8. The ophthalmic agent for the use of claim 6 or 7, wherein the improvement of at least one parameter of visual acuity is selected from an improved accommodation, improved far vision, increased near vision, improved contrast sensitivity, improved stereopsis, and a combination thereof.

9. The ophthalmic agent for the use of any one of claims 1 to 8, wherein the impaired visual acuity is hyperopia, presbyopia, or astigmatism.

10. A composition for use in treating presbyopia, hyperopia, astigmatism, decreased contrast sensitivity or decreased stereopsis in a subject, said composition comprising a methylated xanthine in combination with an ophthalmic miotic agent, wherein the combination is formulated for topical application and applied to an eyelid of the subject and wherein application of the composition to the eyelid of the subject results in a lower incidence of at least one adverse effect associated with application of the miotic agent to the eye, wherein the at least one adverse effect is selected from miosis and myopia.

11. The composition for the use of claim 10, wherein application of the composition to the eyelid of the subject results in improvement of at least one parameter of vision.

12. The composition for the use of claim 11, wherein the improvement in at least one parameter of vison occurs in the substantial absence of miosis or induced myopia.

13. The composition for the use any one of claims 10 to 12, wherein the methylated xanthine is selected from the group consisting of caffeine, theophylline, dyphylline, theobromine, aminophylline, and pentoxifylline and pharmaceutically acceptable salts thereof.

14. The composition for the use of claim 10, wherein the composition comprises 1 to 10 wt% pilocarpine, 1 to 3 wt% carbachol, 4 to 15 wt% cevimeline, or 0.25 to 3 wt% physostigmine.

15. The composition for the use of any one of claims 10 to 14, wherein the methylated xanthine comprises 0.05 to 10 wt% of the composition.

16. The composition for the use of any one of claims 10 to 15, wherein
(i) the methylated xanthine comprises caffeine and the ophthalmic miotic agent comprises carbachol;
(ii) the methylated xanthine comprises caffeine and the ophthalmic miotic agent comprises pilocarpine;
(iii) the methylated xanthine is pentoxifylline and the ophthalmic miotic agent is carbachol;
(iv) the methylated xanthine is pentoxifylline and the ophthalmic miotic agent is pilocarpine; or
(v) the methylated xanthine is pentoxifylline and the ophthalmic miotic agent is physostigmine.

## Patentansprüche

1. Ophthalmisches miotisches Mittel zur Verwendung bei der Behandlung von beeinträchtigter Sehschärfe und der Verringerung des Auftretens von mindestens einem nachteiligen Effekt, der mit der Anwendung des miotischen Mittels auf das Auge eines Subjekts verbunden ist, in Kombination mit mindestens einem methylierten Xanthin oder einem pharmazeutisch verträglichen Salz oder Ester davon, wobei die Kombination zur topischen Anwendung formuliert ist und auf mindestens ein Augenlid des Subjekts aufgetragen wird; und wobei der mindestens eine nachteilige Effekt aus Miosis und Myopie ausgewählt ist.

2. Ophthalmisches miotisches Mittel zur Verwendung nach Anspruch 1, wobei das ophthalmische miotische Mittel Pilocarpin, Carbachol, Cevimelin oder Physostigmin umfasst.

3. Ophthalmisches miotisches Mittel zur Verwendung nach Anspruch 1, wobei die Kombination 1 bis 10 Gew.-% Pilocarpin, 1 bis 3 Gew.-% Carbachol, 4 bis 15 Gew.-% Cevimelin oder 0,25 bis 3 Gew.-% Physostigmin umfasst.

4. Ophthalmisches miotisches Mittel zur Verwendung nach einem der Ansprüche 1 bis 3, wobei das methylierte Xanthin ausgewählt ist aus Koffein, Theophyllin, Dyphyllin, Theobromin, Aminophyllin und Pentoxifyllin und pharmazeutisch verträglichen Salzen davon.

5. Ophthalmisches miotisches Mittel zur Verwendung nach Anspruch 4, wobei das methylierte Xanthin 0,05 bis 10 Gew.-% der Kombination ausmacht.

6. Ophthalmisches miotisches Mittel zur Verwendung nach einem der Ansprüche 1 bis 5, wobei die Kombination zu einer Verbesserung von mindestens einem Parameter der Sehschärfe führt.

7. Ophthalmisches miotisches Mittel zur Verwendung nach Anspruch 6, wobei der mindestens eine Parameter der Sehschärfe für mindestens vier Stunden verbessert wird.

8. Ophthalmisches Mittel zur Verwendung nach Anspruch 6 oder 7, wobei die Verbesserung mindestens eines Parameters der Sehschärfe ausgewählt ist aus einer verbesserten Akkommodation, einer verbesserten Fernsicht, einer erhöhten Nahsicht, einer verbesserten Kontrastempfindlichkeit, einer verbesserten Stereopsis und einer Kombination davon.

9. Ophthalmisches Mittel zur Verwendung nach einem der Ansprüche 1 bis 8, wobei die beeinträchtigte Sehschärfe Hyperopie, Presbyopie oder Astigmatismus ist.

10. Zusammensetzung zur Verwendung bei der Behandlung von Presbyopie, Hyperopie, Astigmatismus, verminderter Kontrastempfindlichkeit oder verminderter Stereopsis eines Subjekts, wobei die Zusammensetzung ein methyliertes Xanthin in Kombination mit einem ophthalmischen miotischen Mittel umfasst, wobei die Kombination zur topischen Anwendung formuliert ist und auf ein Augenlid des Subjekts aufgetragen wird und wobei die Anwendung der Zusammensetzung auf das Augenlid des Subjekts zu einem geringeren Auftreten von mindestens einem nachteiligen Effekt führt, der mit der Anwendung des miotischen Mittels auf das Auge verbunden ist, wobei der mindestens eine nachteilige Effekt aus Miosis und Myopie ausgewählt ist.

11. Zusammensetzung zur Verwendung nach Anspruch 10, wobei die Anwendung der Zusammensetzung auf das Augenlid des Subjekts zu einer Verbesserung mindestens eines Sehparameters führt.

12. Zusammensetzung für die Verwendung nach Anspruch 11, wobei die Verbesserung mindestens eines Sehparameters bei weitgehender Abwesenheit von Miosis oder induzierter Myopie auftritt.

13. Zusammensetzung zur Verwendung nach einem der Ansprüche 10 bis 12, wobei das methylierte Xanthin ausgewählt ist aus der Gruppe, bestehend aus Koffein, Theophyllin, Dyphyllin, Theobromin, Aminophyllin und Pentoxifyllin und pharmazeutisch verträglichen Salzen davon.

14. Zusammensetzung zur Verwendung nach Anspruch 10, wobei die Zusammensetzung 1 bis 10 Gew.-% Pilocarpin, 1 bis 3 Gew.-% Carbachol, 4 bis 15 Gew.-% Cevimelin oder 0,25 bis 3 Gew.-% Physostigmin umfasst.

15. Zusammensetzung zur Verwendung nach einem der Ansprüche 10 bis 14, wobei das methylierte Xanthin 0,05 bis 10 Gew.-% der Zusammensetzung ausmacht.

16. Zusammensetzung zur Verwendung nach einem der Ansprüche 10 bis 15, wobei
(i) das methylierte Xanthin Koffein und das ophthalmische miotische Mittel Carbachol umfasst;
(ii) das methylierte Xanthin Koffein und das ophthalmische miotische Mittel Pilocarpin umfasst;
(iii) das methylierte Xanthin Pentoxifyllin und das ophthalmische miotische Mittel Carbachol ist;
(iv) das methylierte Xanthin Pentoxifyllin und das ophthalmische miotische Mittel Pilocarpin ist; oder
(v) das methylierte Xanthin Pentoxifyllin und das ophthalmische miotische Mittel Physostigmin ist.

## Revendications

1. Agent myotique ophtalmique destiné à une utilisation dans le traitement de l'altération de l'acuité visuelle et la réduction de l'incidence d'au moins un effet indésirable associé à l'application de l'agent myotique sur l'œil d'un sujet, dans une combinaison avec au moins une xanthine méthylée ou un sel ou ester pharmaceutiquement acceptable de cette dernière, dans lequel ladite combinaison est formulée pour une application topique et appliquée au moins sur une paupière du sujet ; et dans lequel le au moins un effet indésirable est sélectionné parmi le myosis et la myopie.

2. Agent myotique ophtalmique destiné à une utilisation selon la revendication 1, dans lequel l'agent myotique ophtalmique comprend de la pilocarpine, du carbachol, de la céviméline ou de la physostigmine.

3. Agent myotique ophtalmique destiné à une utilisation selon la revendication 1, dans lequel la combinaison comprend de 1 à 10 % en poids de pilocarpine, de 1 à 3 % en poids de carbachol, de 4 à 15 % en poids de céviméline ou de 0,25 à 3 % en poids de physostigmine.

4. Agent myotique ophtalmique destiné à une utilisation selon l'une quelconque des revendications 1 à 3, dans lequel la xanthine méthylée est sélectionnée parmi la caféine, la théophylline, la dyphylline, la théobromine, l'aminophylline et la pentoxifylline et des sels pharmaceutiquement acceptables de ces dernières.

5. Agent myotique ophtalmique destiné à une utilisation selon la revendication 4, dans lequel la xanthine méthylée comprend de 0,05 à 10 % en poids de la combinaison.

6. Agent myotique ophtalmique destiné à une utilisation selon l'une quelconque des revendications 1 à 5, dans lequel la combinaison conduit à l'amélioration d'au moins un paramètre de l'acuité visuelle.

7. Agent myotique ophtalmique destiné à une utilisation selon la revendication 6, dans lequel le au moins un paramètre de l'acuité visuelle est amélioré pendant au moins quatre heures.

8. Agent ophtalmique destiné à une utilisation selon la revendication 6 ou 7, dans lequel l'amélioration d'au moins un paramètre de l'acuité visuelle est sélectionné parmi une accommodation améliorée, une vision de loin améliorée, une vision de près accrue, une sensibilité au contraste améliorée, une stéréopsie améliorée et une combinaison de ces dernières.

9. Agent ophtalmique destiné à une utilisation selon l'une quelconque des revendications 1 à 8, dans lequel l'altération de l'acuité visuelle est l'hypermétropie, la presbytie ou l'astigmatisme.

10. Composition destinée à une utilisation dans le traitement de la presbytie, l'hypermétropie, l'astigmatisme, la sensibilité au contraste diminuée ou la stéréopsie diminuée chez un sujet, ladite composition comprenant une xanthine méthylée dans une combinaison avec un agent myotique ophtalmique, dans laquelle la combinaison est formulée en vue d'une application topique et appliquée sur une paupière du sujet et dans laquelle l'application de la composition sur la paupière du sujet conduit à une incidence plus faible d'au moins un effet indésirable associé à l'application de l'agent myotique sur l'œil, dans laquelle le au moins un effet indésirable est sélectionné parmi le myosis et la myopie.

11. Composition destinée à une utilisation selon la revendication 10, dans laquelle l'application de la composition sur la paupière du sujet conduit à l'amélioration d'au moins un paramètre de la vision.

12. Composition destinée à une utilisation selon la revendication 11, dans laquelle l'amélioration d'au moins un paramètre de la vision se produit en l'absence sensible de myosis ou de myopie induite.

13. Composition destinée à une utilisation selon l'une quelconque des revendications 10 à 12, dans laquelle la xanthine méthylée est sélectionnée à partir du groupe constitué par la caféine, la théophylline, la dyphylline, la théobromine, l'aminophylline et la pentoxifylline et des sels pharmaceutiquement acceptables de ces dernières.

14. Composition destinée à une utilisation selon la revendication 10, dans laquelle la composition comprend de 1 à 10 % en poids de pilocarpine, de 1 à 3 % en poids de carbachol, de 4 à 15 % en poids de céviméline ou de 0,25 à 3 % en poids de physostigmine.

15. Composition destinée à une utilisation selon l'une quelconque des revendications 10 à 14, dans laquelle la xanthine méthylée comprend de 0,05 à 10 % en poids de la composition.

16. Composition destinée à une utilisation selon l'une quelconque des revendications 10 à 15, dans laquelle :
(i) la xanthine méthylée comprend de la caféine et l'agent myotique ophtalmique comprend du carbachol ;
(ii) la xanthine méthylée comprend de la caféine et l'agent myotique ophtalmique comprend de la pilocarpine ;
(iii) la xanthine méthylée est de la pentoxifylline et l'agent myotique ophtalmique est du carbachol ;
(iv) la xanthine méthylée est de la pentoxifylline et l'agent myotique ophtalmique est de la pilocarpine ; ou
(v) la xanthine méthylée est de la pentoxifylline et l'agent myotique ophtalmique est de la physostigmine.
